# EUROPEAN PATENT SPECIFICATION

(11) **EP 3 644 943 B1**
(45) Date of publication and mention of the grant of the patent: **11.08.2021**
(21) Application number: 18735276.0
(22) Date of filing: 28.06.2018
(51) Int. Cl.: A61K 8/27, A61K 8/21, A61K 9/00, A61Q 11/00, A61K 8/49, A61K 47/02, A61K 47/10, A61K 47/38, A61K 47/44

(54) **COMPOSITION COMPRISING AN ANTISEPTIC, A VOLATILE SULFUR COMPOUND NEUTRALIZER, AND AN ANTICARIOGENIC AGENT**
ZUSAMMENSETZUNG MIT EINEM ANTISEPTIKUM, EINEM FLÜCHTIGEN SCHWEFELVERBINDUNGSNEUTRALISIERER UND EINEM ANTIKARIOGENEN MITTEL
COMPOSITION COMPRENANT UN ANTISEPTIQUE, UN COMPOSÉ NEUTRALISANT LE SOUFRE VOLATILE ET UN AGENT ANTICARIE

(30) Priority: 29.06.2017 EP 17382414
(43) Date of publication of application: 06.05.2020
(73) Proprietor: Lacer, S.A., 08025 Barcelona (ES)
(72) Inventor: MATA MOLINER, Montserrat, 08025 Barcelona (ES); FERRER SISÓ, Alicia, 08025 Barcelona (ES); FERRÀ DOMINGO, Lourdes, 08025 Barcelona (ES); WIEDEMANN, Ralf, 08025 Barcelona (ES)
(74) Representative: ZBM Patents - Zea, Barlocci & Markvardsen
(86) International application number: PCT/EP2018/067411
(87) International publication number: WO 2019/002462

(56) References cited:
- FR-A1- 3 033 700
- US-A- 4 614 649
- US-A1- 2013 330 116

## Description

### FIELD OF THE INVENTION

The present invention relates to the field of dentistry and oral care, in particular, to a buccal composition comprising octenidine or a salt thereof, a zinc ion source, and a fluoride ion source. It also relates to a process for its preparation, and to its medical use in the treatment of halitosis and other dental problems, as well as to its cosmetic use as an oral care agent.

### BACKGROUND ART

Halitosis also called bad breath or malodor is a condition defined as an unpleasant or offensive odor emanating from the oral cavity, especially, a rear part of the tongue. The major cause of halitosis is the production of Volatile Sulfur Compounds (VSCs) that result from proteolytic degradation of various sulfur-containing substrates in food debris, saliva, blood and cells, by predominantly anaerobic Gram-negative microorganisms (e.g. from the genus *Peptostreptococcus, Eubacterium, Selenomonas, Centipeda, Bacteroides, Fusobacterium, Porphyromonas*) in the oral cavity. Halitosis may also be caused by oral diseases such as tooth decay and periodontitis. Halitosis treatment includes the use of antimicrobial mouthwashes, professional cleaning and the treatment of oral infections and diseases.

Cationic antibacterial agents such as octenidine are well known in the art for their anti-bacterial activity and have been used in oral compositions to control dental plaque and prevent caries and periodontal diseases.

Octenidine has a very wide spectrum of activity and comprises gram-positive and gram-negative bacteria as well as yeasts and fungi. The most common used salt of octenidine is the dihydrochloride salt, whose structure corresponds to formula (I):

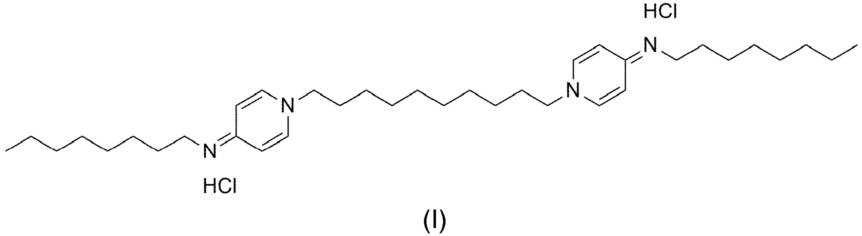

Octenidine has been used as an alternative to other older antiseptic products such as chlorhexidine, in particular with respect to its slow action and concerns about its carcinogenic impurity 4-chloroaniline.

On the other hand, it is well-known, that the frequent use of cationic antibacterial agents in oral hygiene is accompanied by the formation of a cosmetically disfiguring brown stain on the tooth surfaces, being necessary to resort to professionally administered scaling and polishing to remove the stain. It is believed that the staining of teeth arise from an interaction between these agents and certain dietary components, particularly those components rich in tannins such as e.g. tea, coffee and red wine (J Periodontal Res. 1979, 14, pp. 403-410). Although tooth staining is especially pronounced in chlorhexidine-containing products, octenidine has also been reported to lead to undesired staining compared to placebo (J Dent Res. 1990, 69(2), pp. 454-7). FR3033700A1 discloses a buccal cosmetic composition which comprises octenidine and a zinc ion source, together with an orally acceptable carrier.

From what is known in the art it is derived that there is still the need of providing oral stable compositions for the treatment of halitosis and other oral disorders that overcome the problems of the prior art compositions such as dental staining.

### SUMMARY OF THE INVENTION

Inventors have found that a composition that comprises an effective amount of octenidine or a salt thereof, a zinc ion source, and a fluoride ion source shows good stability and antibacterial activity while it minimizes at the same time the staining of the teeth. As demonstrated in the examples below, the composition of the invention surprisingly has less staining side-effects in comparison with a composition containing octenidine and a zinc compound in the absence of a fluoride ion source. As a consequence, the compositions of the invention can be used on a daily basis for long periods of time safely and may perform basic features of oral care such as prevention of dental caries or contribution to tooth care.

Besides, the composition of the invention is able to neutralize Volatile Sulfur Compounds (VSCs) responsible for the bad breadth. And additionally, it also has the required organoleptic properties such as pleasant taste and mouth feel to meet patient compliance.

Thus, a first aspect of the present invention refers to a buccal pharmaceutical, veterinary or cosmetic composition which comprises an effective amount of:
a) octenidine or a pharmaceutically, veterinary or cosmetically acceptable salt thereof,
b) a zinc ion source, and
c) a fluoride ion source,
together with one or more appropriate oral pharmaceutically, veterinary or cosmetically acceptable excipients or carriers.

As mentioned above, thanks to its ability to neutralize Volatile Sulfur Compounds (VSCs), the composition of the invention is useful in the treatment of halitosis. Thus, another aspect of the invention refers to the composition as defined above, for use in the treatment of halitosis.

Due to the presence of an anticariogenic agent the composition of the invention can be used for the prevention of tooth decay or dental caries. Therefore, another aspect of the invention refers to the composition as defined in the first aspect, for use in the prophylaxis of dental caries.

Further, the composition of the invention has also good antimicrobial properties and therefore is also useful for the treatment and prophylaxis of periodontal diseases, gingivitis or dental plaque. Accordingly, another aspect of the invention refers to the composition as defined in the first aspect, for use in the prophylaxis and/or the treatment of periodontal diseases, gingivitis or dental plaque.

In addition, the composition containing octenidine or a salt thereof, a zinc ion source or a fluoride ion source, can also be used for cosmetic purposes in general, for improving dental appearance and/or fresh mouth feeling. Thus, another aspect of the invention relates to the cosmetic use of the composition of the first aspect, as an oral care agent.

### DETAILED DESCRIPTION OF THE INVENTION

All terms as used herein in this application, unless otherwise stated, shall be understood in their ordinary meaning as known in the art. Other more specific definitions for certain terms as used in the present application are as set forth below and are intended to apply uniformly through-out the specification and claims unless an otherwise expressly set out definition provides a broader definition.

The term "percentage (%) by weight" refers to the percentage of each component of the composition in relation to its total weight.

The term "buccal" is used herein to generally refer to the oral cavity, including the buccal mucosa, buccal gingiva, mucous membrane of the tongue, sublingual membrane and the soft palate.

As mentioned above, the first aspect of the invention refers to a buccal pharmaceutical, veterinary or cosmetic composition which comprises an effective amount of:
a) octenidine or a pharmaceutically, veterinary or cosmetically acceptable salt thereof,
b) a zinc ion source, and
c) a fluoride ion source,
together with one or more appropriate oral pharmaceutically, veterinary or cosmetically acceptable excipients or carriers.

For the purposes of the present invention, the term "effective amount" means an amount that is sufficient to obtain the expected effect after its application. In the case of pharmaceutical or veterinary compositions, the effective amount is the "therapeutically effective amount" and refers to the amount of the composition as previously defined that, when administered, is sufficient to prevent development of, alleviate to some extent, one or more of the symptoms of the disorder, disease, or condition being treated. The particular dose of compound administered according to this invention will of course be determined by the particular circumstances surrounding the case, including the specific composition administered, the particular condition being treated, and the similar considerations.

For the purposes of the present invention, the amounts referred to an octenidine salt, to a fluoride ion source or to a zinc ion source refer to the amounts taking into account all components of the whole molecule. For example, when octenidine is present in the composition as octenidine dihydrochloride, the amount referred to the salt includes both the amount of octenidine and the dihydrochloride part. When the zinc ion source is e.g. zinc chloride the amount referred to zinc ion source takes into account also the chloride ion. And when the fluoride ion source is e.g. sodium chloride the amount referred to fluoride ion source takes into account also the sodium ion.

The amounts of octenidine salt, fluoride ion source and zinc ion source can also be expressed as amounts of octenidine (base), zinc ion and fluoride ion without counterparts.

There is no limitation on the type of salt of octenidine that can be used, provided that these are pharmaceutically, veterinary or cosmetically acceptable when they are used. In the context of the present invention, the term "pharmaceutically, veterinary or cosmetically acceptable salt" refers to any non-toxic salt which can be used for therapeutic or cosmetic purposes. The preparation of acceptable salts of octenidine can be carried out by reacting octenidine base with an acid by methods known in the art. Thus, the term "pharmaceutically, veterinary or cosmetically acceptable salts", embraces salts commonly used to form addition salts of free inorganic or organic acids such as e.g. octenidine dihydrochloride, octenidine dihydrobromide, octenidine diacetate, octenidine sulfate, or octenidine disaccharinate.

In one particular embodiment, optionally in combination with one or more features of the various embodiments described above or below, octenidine is used as its dihydrochloride salt. In the present invention, octenidine dihydrochloride and octenidine HCI are used interchangeable, and always refer to the molecule comprising two hydrochloride moieties.

Octenidine or its salt is contained in the composition of the invention in an amount from 0.005 to 0.5% by weight with respect of the total weight of the composition. In one particular embodiment, optionally in combination with one or more features of the various embodiments described above or below, octenidine or its salt is present in an amount from 0.007 to 0.05% by weight, more particularly, from 0.007 to 0.02%, even more particularly in an amount of 0.01 to 0.015% with respect of the total weight of the composition.

The zinc ion source acts as a volatile sulfur compound neutralizer. Generally, the zinc ion source may be a zinc salt such as zinc chloride, zinc acetate, zinc pidolate, zinc lactate, zinc picolinate, zinc ascorbate, zinc citrate, zinc lactate, zinc gluconate, zinc malate, zinc tartrate, zinc carbonate, zinc phosphate, zinc oxide, zinc sulfate or combinations thereof. In one particular embodiment, optionally in combination with one or more features of the various embodiments described above or below, the zinc ion source can be selected from zinc chloride, zinc acetate, zinc pidolate, zinc lactate, and combinations thereof. More particularly, the zinc ion source is zinc chloride.

The zinc ion source is contained in the composition of the invention in an amount from 0.01 to 0.3% by weight with respect of the total weight of the composition. In one particular embodiment, optionally in combination with one or more features of the various embodiments described above or below, the zinc ion source is present in an amount from 0.05 to 0.3% by weight, even more particularly in an amount of 0.1 or 0.25% with respect of the total weight of the composition.

The fluoride ion source acts as anticariogenic agent and is present in the composition of the invention in an effective amount to provide an anti-caries effect. Besides, as shown in the examples it decreases the staining side-effects of octenidine.

Generally, the fluoride ion source may be a fluoride salt or another fluoride containing molecule. Non-limiting examples of fluoride ion sources include sodium fluoride, stannous fluoride, potassium fluoride, indium fluoride, amine fluoride, sodium monofluorophosphate, potassium monofluorophosphate, or combinations thereof or mixtures thereof. In one particular embodiment, optionally in combination with one or more features of the various embodiments described above or below, the fluoride ion source is sodium fluoride.

In one particular embodiment, optionally in combination with one or more features of the various embodiments described above or below, the fluoride ion source is present in an amount from 0.01 to 1% by weight, more particularly, from 0.03 to 0.7%, even more particularly from 0.05 to 0.55% with respect of the total weight of the composition.

Typically, the amount of fluoride ion (without taking into account its counterpart) in the compositions of the invention is equal or lower than 5000 ppm (equivalent to 0.5% w/w), and is preferably comprised from 1000 to 1500 ppm (equivalent to 0.1% to 0.15% w/w).

In another particular embodiment, optionally in combination with one or more features of the various embodiments described above or below, the amount of fluoride ion source expressed as fluoride ion (i.e. without taking into account its counterpart) in the compositions of the invention is equal or lower than 5000 ppm (equivalent to 0.5% w/w), and is preferably comprised from 1000 to 1500 ppm (equivalent to 0.1% to 0.15% w/w).

In one particular embodiment, optionally in combination with one or more features of the various embodiments described above or below, in the composition of the invention the weight ratio between octenidine or a salt thereof and the zinc ion source is comprised from 1:2 to 1:50, more particularly, from 1:5 to 1:30, even more particularly from 1:10 to 1:25.

In another particular embodiment, optionally in combination with one or more features of the various embodiments described above or below, in the composition of the invention the weight ratio between octenidine or a salt thereof and the fluoride ion source is comprised from 1:2 to 1:70, more particularly, from 1:3 to 1:60, even more particularly from 1:5 to 1:55.

In another particular embodiment, optionally in combination with one or more features of the various embodiments described above or below, in the composition of the invention the weight ratio between zinc ion source and the fluoride ion source is comprised from 8:1 to 1:8, more particularly, from 7:1 to 1:7 %, even more particularly from 5:1 to 1:6.

The buccal compositions of the invention can be in liquid, gel, solid, semi-solid or pasty form. Liquid compositions include, but are not limited to mouthwash, a mouthrinse, an oral solution, a dispersion through a water jet, spray, a breath freshener, and gargle. Solid, semi-solid or pasty compositions include a toothpaste, a dentifrice, a tooth powder, dental cream, gummies, lozenges, candies, strips, tablets or capsules.

In one particular embodiment, optionally in combination with one or more features of the various embodiments described above or below, the buccal composition of the invention is in the form of a toothpaste, a mouthwash or a spray.

In one particular embodiment, optionally in combination with one or more features of the various embodiments described above or below, the composition of the invention is a mouthwash or a spray, and the fluoride ion source is comprised in an amount from 0.02 to 0.15% by weight, more particularly, from 0.03 to 0.07%, even more particularly is 0.05 with respect to the total weight of composition.

In another particular embodiment, optionally in combination with one or more features of the various embodiments described above or below, the composition of the invention is a mouthwash or a spray, and the weight ratio between octenidine or a salt thereof, and the fluoride ion source is from 1:2 to 1:10, more particularly, from 1:3 to 1:8, even more particularly is 1:5 or 2:5.

In another particular embodiment, optionally in combination with one or more features of the various embodiments described above or below, the composition of the invention is a mouthwash or a spray, and the weight ratio between the zinc ion source, and the fluoride ion source is from 1:1 to 7:1, more particularly, from 2:1 to 6:1, even more particularly is 2:1 or 5:1.

In another particular embodiment, optionally in combination with one or more features of the various embodiments described above or below, the composition of the invention is a mouthwash or a spray, and the weight ratio between octenidine or a salt thereof, the zinc ion source, and the fluoride ion source is 1:10:5, 2:10:5, 1:25:5 or 1:25:5.

In another particular embodiment, optionally in combination with one or more features of the various embodiments described above or below, the composition of the invention is a toothpaste, and the fluoride ion source is comprised in an amount from 0.2 to 1% by weight, more particularly, from 0.3 to 0.7%, even more particularly is 0.55 with respect to the total weight of composition.

In another particular embodiment, optionally in combination with one or more features of the various embodiments described above or below, the composition of the invention is a toothpaste, and the weight ratio between octenidine or a salt thereof, and the fluoride ion source is from 1:20 to 1:70, more particularly, from 1:25 to 1:60, even more particularly is 1:55 or 2:55.

In another particular embodiment, optionally in combination with one or more features of the various embodiments described above or below, the composition of the invention is a toothpaste, and the weight ratio between the zinc ion source, and the fluoride ion source is from 1:1 to 1:7, more particularly, from 1:2 to 1:6, even more particularly is 1:5.5 or 2.5:5.5.

In another particular embodiment, optionally in combination with one or more features of the various embodiments described above or below, the composition of the invention is a toothpaste, and the weight ratio between octenidine or a salt thereof, the zinc ion source, and the fluoride ion source is 1:10:55, 2:10:55, 1:25:55 or 1:25:55.

The compositions of the invention in the form of pastes/emulsions show phase stability. For the purposes of the invention, the terms "phase stability" means that the composition can resist syneresis, i.e., no liquid separation from the composition is observed visually over a defined period of time under ambient conditions.

The compositions of the invention are stable under storage conditions, i.e. show shelf-life stability. For the purposes of the invention, the term "shelf-life stability" means that the oral care compositions are acceptable for consumption after a defined period of time after its production under ambient conditions.

Generally, the compositions of the invention have at least more than 24 months, and more particularly more than 36 months of shelf-life stability and/or phase stability.

Additionally, the buccal compositions disclosed herein may optionally comprise, in addition to the composition of the invention described above, further active ingredients. Non-limiting examples of further active ingredients include, without limitation, further antimicrobial agents, such as for example, chlorhexidine or PVP-iodine; further antiplaque agents, such as triclosan, hexetidine, cetylpiridinium chloride; further anticariogenic agents, such as xylitol, Manuka honey and tannin; periodontal tissue regenerating agents, such as allantoine, aloe vera, enoxolone, pantenol, and tocopheryl acetate; hydratants such as trehalose, betaine and azulene, and mixtures thereof. When further active agents are present in the compositions of the invention they are present in an effective amount as defined above.

The buccal compositions of the invention also comprise one or more appropriate pharmaceutically, veterinary or cosmetically acceptable excipients or carriers. The expression "pharmaceutically or veterinary acceptable excipients or carriers" refers to pharmaceutically or veterinary acceptable materials, compositions or vehicles for use in the pharmaceutical/veterinary technology for preparing compositions with medical/veterinary use. Each component must be pharmaceutically acceptable in the sense of being compatible with the other ingredients of the pharmaceutical/veterinary composition. It must also be suitable for use in contact with the mouth, tongue and teeth of animals, including humans, without excessive toxicity, irritation, allergic response, or other problems or complications commensurate with a reasonable benefit/risk ratio.

The expression "cosmetically acceptable excipients or carriers" refers to that excipients or carriers suitable for use in contact with animal or human mouth, tongue and teeth without undue toxicity, incompatibility, instability, allergic response, among others.

The choice of the suitable excipients and/or carriers, and their amounts, may be readily determined by those skilled in the art in accordance to the type of formulation prepared and considering important aspects such as chemical compatibility. In particular, such selection of excipients and/or carriers should not interfere with the effect of the combination of octenidine or its salt, a zinc ion source, and a fluoride salt.

The excipients of the compositions of the invention can be selected from binders, diluents, chelating agents, disintegrants, thickening or film-forming agents, humectant, sweeteners, abrasive agents, flavoring agents, masking agents, buffering agents, cooling agents, coloring agents, surfactants, preservatives, lubricants, opacifiers and solvents.

Non-limiting examples of binders include, without limitation, starch, pregelatinized starch, sodium carboxymethylcellulose, hydroxypropyl cellulose, hydroxy propyl methylcellulose, methyl cellulose, polyvinyl pyrrolidone, polyethylene glycol, hydrolyzed gelatin, natural gums, or combinations thereof.

Non-limiting examples of diluents include, without limitation, starch, microcrystalline cellulose, lactose, maltose, fructose, guar gum, magnesium hydroxide, dicalcium phosphate, and the like or any combinations thereof.

Non-limiting examples of chelating agents include, without limitation, alkali metal salts of tartaric acid and citric acid, alkali metal salts of pyrophosphates or polyphosphates, sodium benzoate, butylated hydroxytoluene, butylated hydroxyanisole, ethylenediaminetetraacetic acid, sodium gluconate, or combinations thereof.

Non-limiting examples of disintegrants include, without limitation, natural, modified or pregelatinized starch, crospovidone, croscarmellose sodium, sodium starch glycolate, low-substituted hydroxypropyl cellulose, or combinations thereof.

Non-limiting examples of thickening agents include, without limitation, carboxymethyl cellulose, gum tragacanth, gum arabic, gum Karaya, sodium alginate, hydroxyethyl cellulose, methyl and ethyl cellulose, carrageenan, xanthan gum, polyvinyl pyrrollidone, silica aerogels, thickening silica, or combinations thereof.

Non-limiting examples of humectants include, without limitation, trehalose, polyethylene glycol, propylene glycol, glycerin (glycerol), hydrogenated starch hydrolyzates, or mixtures thereof.

Non-limiting examples of sweeteners include, without limitation, sucrose, glucose, fructose, maltose, maltodextrin, sodium saccharin, calcium cyclamate, aspartame (N-L-alpha- aspartyl-L-phenylalanine methyl ester), neohesperidin, acesulfame potassium, sucralose, dihydrochalcone, glycyrrhizin and glycyrrhizin salts, sugar alcohols, such as mannitol, sorbitol, xylitol, maltitol, or combinations thereof.

Non-limiting examples of abrasive agents include, without limitation, silica, or combinations thereof.

Non-limiting examples of flavoring agents include aroma, essential oils of spearmint, peppermint, wintergreen, sassafras, clove, sage, eucalyptus, marjoram, cinnamon, lemon, lime, grapefruit and orange, menthol, eucalyptol, thymol, carvone and anethole.

Non-limiting examples of masking agent include, without limitation, anethole, dihydroanethole, eugenol, vanillin, ethylvanillin, ethyl maltol, neohesperidin dihydrochalcone.

Non-limiting examples of buffering agents include, without limitation, monosodium phosphate, trisodium phosphate, sodium hydroxide, potassium hydroxide, alkali metal carbonate salts, sodium carbonate, imidazole, pyrophosphate salts, citric acid, sodium citrate, or combinations thereof. Non-limiting examples of cooling agents include, without limitation, menthone glycerol acetal, menthyl lactate, menthyl ethyl oxalate, menthol and menthol derivatives.

Non-limiting examples of coloring agents include, without limitation, a dye, an aluminum lake, iron oxides, or combinations thereof.

Non-limiting examples of surfactants include, without limitation, Cocamidopropyl betaine, sodium lauryl sulfate, glycerol fatty acid esters; acetylated glycerol fatty acid esters; propylene glycol fatty acid esters; sorbitan fatty acid esters, PEG-40 hydrogenated castor oil, PEG-40 sorbitan diisostearate; taurine, sarcosine, sodium N-methyl-N-palmitoyl taurinate, sodium N-lauryl or sarcosinate, sodium N- myristyl sarcosinate, sodium N-palmitoyl sarcosinate, or combinations thereof.

Non-limiting examples of preservatives include citric acid, sodium benzoate, cetylpyridinium chloride, potassium sorbate, alkyl parabens, or combinations thereof.

Non-limiting examples of lubricants include, without limitation, magnesium stearate, calcium stearate, high molecular weight polyethylene glycol, stearic acid, talc, and sodium stearyl fumarate, or mixtures thereof.

Non-limiting examples of opacifiers include, without limitation, titanium dioxide, cerium oxide (CeO₂), tin oxide (SnO₂), zirconium dioxide (ZrO₂), or combinations thereof.

Non-limiting examples of solvents include, without limitation, water, propylene glycol, ethyl alcohol or combinations thereof.

Typically a mouthwash according to the invention may comprise one or more of solvents (from 40% to 90%), humectants (from 0% to 10%), surfactants (from 0% to 5%), flavoring agents (from 0.2% to 1%), sweetening agents (from 0.01% to 1%), thickening agents (from 0% to 0.5%), buffers (from 0% to 1%), cooling agents (from 0% to 0.3%), masking agents (from 0% to 1%), and coloring agents (from 0% to 0.1%).

Typically a spray according to the invention may comprise one or more of solvents (from 40% to 90%), humectants (from 0% to 10%), surfactants (from 0% to 5%), flavoring agents (from 0.2% to 1%), sweetening agents (from 0.01% to 1%), thickening agents (from 0% to 0.5%), buffers (from 0% to 1%), cooling agents (from 0% to 0.3%), and masking agents (from 0% to 1%). Typically a toothpaste according to the invention may comprise one or more of solvents (from 5% to 40%), humectants (from 10% to 60%), surfactants (from 1% to 5%), flavoring agents (from 0.01% to 0.1%), sweetening agents (from 0.1% to 0.5%), thickening agents (from 8% to 20%), buffers (from 0% to 5%), cooling agents (from 0% to 0.2%), masking agents (from 0% to 0.01%), chelating agents (from 0% to 0.1%), abrasive agents (from 1% to 5%), and coloring agents (from 0% to 0.1%).

The solid buccal compositions of the invention may be prepared according to methods well known in the art, for example by physical admixture, wet granulation, dry granulation, and/or direct compression.

As mentioned above, the composition of the invention is useful in the treatment of halitosis, thanks to its ability to neutralize Volatile Sulfur Compounds (VSCs).

For the purposes of the invention, the term "treatment" of the disease refers to stopping or delaying of the disease progress, when the composition of the invention is used in the subject exhibiting symptoms of disease onset. The term "prevention" refers to stopping or delaying of symptoms of disease onset, when the composition of the invention is used in the subject exhibiting no symptoms of disease onset but having high risk of disease onset.

In one particular embodiment, optionally in combination with one or more features of the various embodiments described above or below, the composition of the invention for use in halitosis is a pharmaceutical or veterinary composition. In another particular embodiment, optionally in combination with one or more features of the various embodiments described above or below, the composition of the invention for use in halitosis is a cosmetic composition.

The invention also refers to the use of an effective amount of a) octenidine or a pharmaceutically, veterinary or cosmetically acceptable salt thereof, b) a zinc ion source, and c) a fluoride ion source, for the manufacture of a buccal pharmaceutical, veterinary or cosmetic composition as defined above, for the treatment of halitosis. It also forms part of the invention a method for the treatment of halitosis, comprising buccally administering an effective amount of the composition as defined in the first aspect, in a subject in need thereof, including a human.

Further, since the composition of the invention has also good antimicrobial properties it is useful for the treatment and prophylaxis of periodontal diseases, and the prophylaxis of dental caries.

In one particular embodiment, optionally in combination with one or more features of the various embodiments described above or below, the composition of the invention for use in the treatment and prophylaxis of periodontal diseases, and the prophylaxis of dental caries is a pharmaceutical or veterinary composition. In another particular embodiment, optionally in combination with one or more features of the various embodiments described above or below, the composition of the invention for use in the treatment and prophylaxis of periodontal diseases, and the prophylaxis of dental caries is a cosmetic composition.

Accordingly, the invention also refers to the use of a) octenidine or a pharmaceutically, veterinary or cosmetically acceptable salt thereof, b) a zinc ion source, and c) a fluoride ion source, for the manufacture of a buccal pharmaceutical, veterinary or cosmetic composition as defined above, for the prophylaxis and/or the treatment of periodontal diseases, gingivitis or dental plaque. It also forms part of the invention a method for the prophylaxis and/or the treatment of periodontal diseases, gingivitis or dental plaque, comprising buccally administering an effective amount of the composition as defined in the first aspect, in a subject in need thereof, including a human.

The invention also relates to the use of a) octenidine or a pharmaceutically, veterinary or cosmetically acceptable salt thereof, b) a zinc ion source, and c) a fluoride ion source, the composition as defined in the first aspect, for the manufacture of a buccal pharmaceutical, veterinary or cosmetic composition as defined above, for the prophylaxis of dental caries. It also forms part of the invention a method for the prophylaxis of dental caries, comprising buccally administering an effective amount of the composition as defined in the first aspect, including a human.

In addition, the composition of the invention can also be used for cosmetic purposes in general, for improving dental appearance and/or fresh mouth feeling. Thus, it also forms part of the invention a cosmetic method for oral care of a subject in need thereof, including a human, said method comprising administering to said subject, including a human, an effective amount of the composition of the first aspect, together with cosmetically acceptable excipients or carriers.

The term "cosmetic" is intended to denote a use intended, principally, to provide an aesthetic and/or comfort effect, in particular, to ameliorate the appearance of the teeth, and/or fresh mouth feeling. In the context of the present invention, when the composition of the invention is used as an oral care agent it does not intend to include any therapeutic use. The cosmetic use is addressed particularly to human beings that do not have any dental problems or diseases as e.g. halitosis, periodontal diseases, caries or tooth decay, and the like. When the composition of the invention is used for cosmetic purposes, it forms part of a topical cosmetic composition.

Throughout the description and claims the word "comprise" and variations of the word, are not intended to exclude other technical features, additives, components, or steps. Furthermore, the word "comprise" encompasses the case of "consisting of". Additional objects, advantages and features of the invention will become apparent to those skilled in the art upon examination of the description or may be learned by practice of the invention. The following examples are provided by way of illustration, and they are not intended to be limiting of the present invention. Furthermore, the present invention covers all possible combinations of particular and preferred embodiments described herein.

### EXAMPLES

### Buccal compositions

### Example 1: Mouthwash compositions

### Example 1.1

A mouthwash composition according to this invention was prepared having the following components:

| **Group** | **Name** | **% (w/w)** |
|---|---|---|
| 1 | hydroxyethylcellulose | 0.050 |
| 2 | zinc chloride | 0.100 |
| 2 | sodium fluoride | 0.050 |
| 2 | sweetener | 1.120 |
| 2 | buffer | q.s. pH 5.6 (± 0.3) |
| 2 | glycerin | 5.000 |
| 3 | octenidine HCl | 0.010 |
| 3 | PEG-hydrogenated castor oil | 2.000 |
| 3 | propylene glycol | 7.000 |
| 3 | flavoring | 0.593 |
| | aqua | q.s. 100 mL |

The above composition was prepared following a process including the following steps:
1. In a reactor of sufficient capacity, hydroxyethylcellulose and water were introduced and stirred for 20 min.
2. All the ingredients of the group 2 were successively added to the reactor.
3. The mixture was stirred for 30 min.
4. The ingredients of group 3 were dissolved together (by stirring them for 30 min) and the resulting solution was added to the reactor.
5. The mixture was stirred for 30 min.
6. The pH was checked and adjusted to 5.4-5.8 with citric acid and the mixture was stirred for 30 min. The final solution was filtered.

### Example 1.2:

A mouthwash having the composition shown below was prepared following the process as described in example 1.1.

| **Group** | **Name** | **% (w/w)** |
|---|---|---|
| 1 | hydroxyethylcellulose | 0.050 |
| 2 | zinc chloride | 0.100 |
| 2 | sodium fluoride | 0.050 |
| 2 | sweetener | 1.120 |
| 2 | buffer | q.s. pH 5.6 (± 0.3) |
| 2 | glycerin | 5.000 |
| 3 | octenidine HCl | 0.020 |
| 3 | PEG hydrogenated castor oil | 2.000 |
| 3 | propylene glycol | 7.000 |
| 3 | flavoring | 0.593 |
| | aqua | q.s. 100 mL |

### Example 1.3:

A mouthwash having the composition shown below was prepared following the process as described in example 1.1.

| **Group** | **Name** | **% (w/w)** |
|---|---|---|
| 1 | hydroxyethylcellulose | 0.050 |
| 2 | zinc chloride | 0.250 |
| 2 | sodium fluoride | 0.050 |
| 2 | sweetener | 1.120 |
| 2 | buffer | q.s. pH 5.6 (± 0.3) |
| 2 | glycerin | 5.000 |
| 3 | octenidine HCl | 0.010 |
| 3 | PEG hydrogenated castor oil | 2.000 |
| 3 | propylene glycol | 7.000 |
| 3 | flavoring | 0.593 |
| | aqua | q.s. 100 mL |

### Comparative example 1.4 (without sodium fluoride):

A mouthwash having the composition shown below was prepared following the process as described in example 1.1.

| **Group** | **Name** | **% (w/w)** |
|---|---|---|
| 1 | hydroxyethylcellulose | 0.050 |
| 2 | zinc chloride | 0.100 |
| 2 | sweetener | 1.120 |
| 2 | buffer | q.s. pH 5.6 (± 0.3) |
| 2 | glycerin | 5.000 |
| 3 | octenidine HCl | 0.010 |
| 3 | PEG hydrogenated castor oil | 2.000 |
| 3 | propylene glycol | 7.000 |
| 3 | flavoring | 0.593 |
| | aqua | q.s. 100 mL |

### Comparative example 1.5 (without zinc chloride):

A mouthwash having the composition shown below was prepared following the process as described in example 1.1.

| **Group** | **Name** | **% (w/w)** |
|---|---|---|
| 1 | hydroxyethylcellulose | 0.050 |
| 2 | sodium fluoride | 0.050 |
| 2 | sweetener | 1.120 |
| 2 | buffer | q.s. pH 5.6 (± 0.3) |
| 2 | glycerin | 5.000 |
| 3 | octenidine HCl | 0.010 |
| 3 | PEG hydrogenated castor oil | 2.000 |
| 3 | propylene glycol | 7.000 |
| 3 | flavoring | 0.593 |
| | aqua | q.s. 100 mL |

### Example 2: Toothpaste composition

A toothpaste composition of the invention was prepared having the following components:

| **Group** | **Name** | **% (w/w)** |
|---|---|---|
| 1 | hydrogenated starch hydrolysate | 46.2000 |
| 2 | buffer | 0.4000 |
| 2 | sodium fluoride | 0.5520 |
| 2 | sodium gluconate | 0.0500 |
| 2 | sweetener | 1.27000 |
| 3 | glycerin | 6.0000 |
| 3 | xanthan gum | 0.5000 |
| 4 | octenidine HCl | 0.0100 |
| 4 | propylene glycol | 0.5000 |
| 5 | silica | 14.0000 |
| 5 | zinc chloride | 0.1000 |
| 6 | Colorant | 0.0003 |
| 7 | flavoring | 1.3045 |
| 8 | cocamidopropyl betaine | 0.6000 |
| | aqua | q.s. 100g |

The above composition was prepared by a process including the following steps:
1. Hydrogenated starch hydrolysate and water were introduced into a reactor of sufficient capacity, and stirred for 5 min.
2. All ingredients of group 2 of the table were successively added, stirred and homogenized or dispersed (turrax) for 10 min.
3. The ingredients of group 3 were dissolved together and added to the reactor under stirring, and vacuum (-0.9 bar) for 20 min.
4. The ingredients of group 4 were dissolved together and added to the reactor, and the dispersion was stirred under vacuum for 30 min.
5. The ingredients of group 5 were added to the reactor successively to the reactor, and the dispersion was stirred under vacuum.
6. Coloring was added to the reactor.
7. The ingredients of group 7 were dissolved and added to the reactor, and the dispersion was stirred under vacuum for 15 min
8. Finally, cocamidopropyl betaine was added to the reactor, and the dispersion was stirred under vaccum for 30 min.

### Example 3: Spray composition

A spray composition according to the invention was prepared having the following components:

| **Group** | **Name** | **% (w/w)** |
|---|---|---|
| 1 | hydroxyethyl cellulose | 0.050 |
| 2 | zinc chloride | 0.100 |
| 2 | sodium fluoride | 0.050 |
| 2 | sweetener | 1.120 |
| 2 | buffer | q.s. pH 5.6 (± 0.3) |
| 2 | glycerin | 5.000 |
| 3 | octenidine HCl | 0.010 |
| 3 | PEG hydrogenated castor oil | 2.000 |
| 3 | propylene glycol | 7.000 |
| 3 | flavoring | 0.654 |
| | aqua | q.s.100 mL |

The above composition was prepared following the process as described in example 1.1.

### Microbiological tests

In order to determine the antimicrobial activity of the compositions of invention, two types of tests were performed:
- *In vitro* studies of oral solutions according to the invention in front of single bacterial species
- *In vitro* studies of oral mouthwashes according to the invention in front multispecies biofilm

### In vitro studies in front of single bacterial species

The Minimum Inhibitory Concentration (MIC) and Minimum bactericidal concentration (MBC) of the compositions of examples 1.1 and 1.2 were determined on single bacterial species separately in: *Escherichia coli; Enterococcus faecalis, Candida albicans, Streptococcus mutans,* and *Streptococcus oralis.*

MIC was determined using a standar microdilution method according to. EUCAST (European Committee on Antimicrobial Susceptibility Testing) and CLSI (Clinical and Laboratory Standards Institute) (Clin Microbiol Infect 2006, 501-503; Clin Microbiol Infect 2003, ix-xv; Clin Microbiol Infect 2000, 503-508; J Pharm Analysis 2016, 6, 71-79). For each tested sample a dilution bank was prepared and for each microorganism and the value of colony forming units per milliliter (cfu/mL) of a culture was determined. The inoculum was adjusted following the guidelines and a determined concentration was introduced at each dilution. MIC values correspond to minimum concentrations of sample capable of completely inhibiting the visible growth of microorganisms.

MBC were determined using a standard dilution method according to EUCAST (European Committee on Antimicrobial Susceptibility Testing) and CLSI (Clinical and Laboratory Standards Institute) (Clin Microbiol Infect 2006, 501-503; Clin Microbiol Infect 2000, 503-508; J Pharm Analysis 2016, 6, 71-79), 100 µL aliquots were seeded in solid media from the previous dilutions to the MIC value. Plates were incubated under suitable growth conditions, according to the species involved. The MBC of each tested sample, for each study microorganism, corresponds to the minimum concentration of sample capable of completely inhibiting microbial growth.

The obtained results are shown in following tables:

| | **MIC** | | | | |
|---|---|---|---|---|---|
| Tested sample | *E. coli* | *E. faecalis* | *C. albicans* | *S. mutans* | *S.oralis* |
| Example 1.1 | 1/128 | 1/512 | 1/512 | 1/1024 | 1/256 |
| Example 1.2 | 1/256 | 1/512 | 1/256 | 1/1024 | 1/512 |

| | **MBC** | | | | |
|---|---|---|---|---|---|
| Tested sample | *E. coli* | *E. faecalis* | *C. albicans* | *S. mutans* | *S.oralis* |
| Example 1.1 | 1/16 | 1/16 | 1/2 | 1/128 | 1/16 |
| Example 1.2 | 1/16 | 1/8 | 1/32 | 1/256 | 1/64 |

No significant differences were observed between the two concentrations of octenidine tested. Both compositions showed good MIC and MBC.

### In vitro studies in front multispecies biofilm

To establish antimicrobial activity, *in vitro* bacterial viability assays were performed on multispecies biofilms of anaerobic microorganisms associated with halitosis, in particular, Aerotolerant Anaerobic Bacteria (*Streptococcus oralis*)*,* and Strict anaerobic bacteria (*Fusobacterium nucleatum* and *Prevotella intermedia*)*.*

The determination of the effect of the composition of example 1.1 on the bacterial viability of microorganisms grown in biofilm was carried out according to the following experimental procedure:
1. The concentrations of the bacterial suspensions were adjusted by the use of atomic force microscopy (AFM)
2. Once the concentrations were adjusted, a multispecies bacterial suspension was prepared and inoculated into Costar 96 well plates.
3. Plates were incubated at 37 °C under anaerobic conditions for 96 hours.
4. Once the biofilm was formed, the bacteria were planktonically removed by washing with a 1/4 Ringer solution.
5. The treatment wells were filled with the test sample and incubated for 2-3 minutes.
6. After this time, the wells were washed with Ringer 1/4 to remove test sample residues and the microbial viability of the biofim was quantified with the CTC technique (5-cyano-2,3-ditolyl tetrazolium chloride)
7. CTC is used as a red-dox indicator of the microbial metabolic activity of aerotolerant and anaerobic bacteria.
8. Reduction of CTC in all phases of bacterial growth generates a highly insoluble reddish and highly fluorescent compound. The detection and quantification of this compound by absorbance (450 nm) or by fluorescence (excitation peak: 480 nm; emission peak: 630 nm) allows the determination of microbial viability after a disinfection process.

The obtained results are shown in following table:

| **Sample** | **% microbial viability** |
|---|---|
| Octenidine 0.01% + Zinc chloride 0.1% | 23.23 |

This result show that the above composition decreased significantly the percentage of bacteria involved in halitosis disease. Accordingly, the compositions of the invention are suitable as a method of oral hygiene for the maintenance of a normal oral microbiota and the prevention of halitosis.

### Sulfur neutralization

In order to estimate the action of the compositions of the invention over sulfur-derived compounds (VSC's: volatile sulfur compounds) responsible for malodor in halitosis, the percentage of hydrogen sulfide (H₂S) neutralized was measured from the combination of 1 mL of a stock solution of H₂S (0.6 mg / mL) with 5 mL of a mouthwash composition according to the inventions.

Quantification was performed by the mean absorbance of the resulting solution at 288 nm in triplicate. The quantification was done by interpolation in a calibration line:
- Abs 288 nm = 2.0065 - 1.51476 * mg Sulfides
- Correlation coefficient = -0.996725

The obtained results are shown in following table:

| **Sample** | **% SH₂ neutralized** |
|---|---|
| Comparative example 1.5 | 0 |
| Example 1.1 | 54.2 |
| Example 1.3 | 66.5 |

The results show that the compositions containing a zinc salt were very efficient in increasing the percentage of SH₂ neutralized.

### Staining study

The method is based on the in vitro method described by Addy (1979) to evaluate the dental staining produced by the adsorption of colored substances from an infusion of tea on an acrylic support due to the presence of cationic antiseptics as chlorhexidine digluconate or octenidine dihydrochloride.

The experimental method used was the following:
1. Acrylic pieces (PMMA (Perspex), 30 mm x 10 mm x 6 mm) were built to fit in the Spectrophotometer cell.
2. The pieces were exposed to different solutions (1% tea Solution) during controlled time (60 min) at 37°C.
3. Tested samples were added to the solution above at 37 °C for 3-5 min. Water was used as a negative control, and chlorhexidine digluconate 0.12% w/w mouthwash as a positive control.
4. After that, test samples were washed with water (3-5 minutes)
5. Finally, test sample were dried at 50 °C (5 min)

Every acrylic piece was exposed to the all process above steps 1-5 six times. After that, the absorbance at 395 nm of every piece was determined with a UV-VIS-spectrophotometer. The value for chlorhexidine was set at 100%. The obtained results are shown in following table:

| **Sample** | **Staining** |
|---|---|
| Chlorhexidine 0.12% | 100% |
| Comparative example 1.4 | 17.7% |
| Example 1.1 | 3.7% |
| Water | 15.5% |

The results showed that the combination of octenidine and zinc salt without NaF almost had the same effect in this staining *in vitro* model than water (negative control). Surprisingly, the presence, of sodium fluoride decreased significantly the percentage of staining, even below the value of the negative control (water).

### Stability studies

Stability tests were performed with the sample of example 1.1 under the following temperature-controlled conditions:
- At 25 °C, 30 °C, 40 °C (1, 3 and 6 months)
- At 50 °C (1 month)
- At 25 °C and 30 °C (9, 12, 18, 24 and 36 months).

At the time of 1, 3 and 6 months, physical, chemical/organoleptic and chemical (component analysis) controls were performed. Microbiological tests were performed only at 40 °C (6 months) and 50 °C (1 month) tests. According to the obtained results it was seen that the formulation of the invention was stable under the tested conditions.

### REFERENCES CITED IN THE APPLICATION

Addy M. et al., "An in vitro study of the role of dietary factors in the aetiology of tooth staining associated with the use of chorhexidine", J. Periodontal Res. 1979, 14, pp. 403-10
Beiswanger BB et al, "The clinical effects of a mouthrinse containing 0.1% octenidine", J Dent Res. 1990, 69(2), pp. 454-7
Kahlmeter G. et al, "European Committee on Antimicrobial Susceptibility Testing (EUCAST) Technical Notes on antimicrobial susceptibility testing", Clinical Microbiology and Infection 2006, vol 12, number 6, pp. 501-3
"Determination of minimum inhibitory concentrations (MICs) of antibacterial agents by broth dilution", Clinical Microbiology and Infection 2003, vol. 9, number 8, pp. ix-xv
"Terminology relating to methods for the determination of susceptibility of bacteria to antimicrobial agents", Clinical Microbiology and Infection 2000, vol. 6, number 9, pp. 503-508
Balouiri M. et al., "Methods for in vitro evaluating antimicrobial activity: A review", Journal of Pharmaceutical Analysis 2016, 6, 71-79

## Claims

1. A buccal pharmaceutical, veterinary or cosmetic composition which comprises an effective amount of:
a) octenidine or a pharmaceutically, veterinary or cosmetically acceptable salt thereof,
b) a zinc ion source, and
c) a fluoride ion source,
together with one or more appropriate oral pharmaceutically, veterinary or cosmetically acceptable excipients or carriers.

2. The composition according to claim 1, wherein octenidine is present as octenidine dihydrochloride.

3. The composition according to any of the claims 1-2, wherein octenidine or its salt is comprised in an amount from 0.005 to 0.5% by weight with respect to the total weight of composition.

4. The composition according to any of the claims 1-3, wherein the zinc ion source is selected from zinc acetate, zinc chloride, zinc pidolate, zinc lactate and combinations thereof.

5. The composition according to any of the claims 1-4, wherein the zinc ion source is comprised in an amount from 0.01 to 0.3% by weight with respect to the total weight of composition.

6. The composition according to any of the claims 1-5, wherein the fluoride ion source is sodium fluoride.

7. The composition according to any of the claims 1-6, wherein the fluoride ion source is comprised in an amount from 0.01 to 1% by weight with respect to the total weight of composition.

8. The composition according to any of the claims 1-7, wherein the excipients or carriers are selected from the group consisting of binders, diluents, chelating agents, disintegrants, thickening or film-forming agents, humectant, sweeteners, abrasive agents, flavoring agents, masking agents, buffering agents, cooling agents, coloring agents, surfactants, preservatives, lubricants, opacifiers, and solvents.

9. The composition according to any of the claims 1-8, which further comprises one or more additional active ingredients selected from antimicrobial agents, antiplaque agents, anticariogenic agents, periodontal tissue regenerating agents and hydratants.

10. The composition according to any of the claims 1-9, which is a mouthwash, a mouthrinse, a dentifrice, a toothpaste, a tooth powder, an oral solution, a spray, a dental gel, a dispersion through a water jet, a breath freshener, or a gargle.

11. The composition according to any of the claims 1-10, which is a mouthwash or a spray and wherein the fluoride ion source is comprised in an amount from 0.02 to 0.15% by weight with respect to the total weight of composition.

12. The composition according to any of the claims 1-10, which is a toothpaste wherein the fluoride ion source is comprised in an amount from 0.2 to 1% by weight with respect to the total weight of composition.

13. A composition as defined in any of the claims 1-12, for use in the treatment of halitosis, or in the prophylaxis of dental caries.

14. A composition as defined in any of the claims 1-12, for use in the prophylaxis and/or the treatment of periodontal diseases, gingivitis or dental plaque.

15. Cosmetic use of a cosmetic composition as defined in any of the claims 1-12, as an oral care agent.

## Patentansprüche

1. Eine bukkale pharmazeutische, tiermedizinische oder kosmetische Zusammensetzung, die eine wirksame Menge umfasst von:
a) Octenidin oder ein pharmazeutisch, tiermedizinisch oder kosmetisch akzeptables Salz davon,
b) eine Zinkionenquelle und
c) eine Fluoridionenquelle,
zusammen mit einem oder mehreren geeigneten oralen pharmazeutisch, tiermedizinisch oder kosmetisch akzeptablen Hilfsstoffen oder Trägersubstanzen.

2. Die Zusammensetzung nach Anspruch 1, wobei das Octenidin als Octenidindihydrochlorid vorliegt.

3. Die Zusammensetzung nach einem der Ansprüche 1 bis 2, wobei das Octenidin oder sein Salz in einer Menge von 0,005 bis 0,5 Gew.-% bezogen auf das Gesamtgewicht der Zusammensetzung enthalten ist.

4. Die Zusammensetzung nach einem der Ansprüche 1 bis 3, wobei die Zinkionenquelle ausgewählt ist aus Zinkacetat, Zinkchlorid, Zinkpidolat, Zinklactat und Kombinationen davon.

5. Die Zusammensetzung nach einem der Ansprüche 1 bis 4, wobei die Zinkionenquelle in einer Menge von 0,01 bis 0,3 Gew.-% bezogen auf das Gesamtgewicht der Zusammensetzung enthalten ist.

6. Die Zusammensetzung nach einem der Ansprüche 1 bis 5, wobei die Fluoridionenquelle Natriumfluorid ist.

7. Die Zusammensetzung nach einem der Ansprüche 1 bis 6, wobei die Fluoridionenquelle in einer Menge von 0,01 bis 1 Gew.-% bezogen auf das Gesamtgewicht der Zusammensetzung enthalten ist.

8. Die Zusammensetzung nach einem der Ansprüche 1 bis 7, wobei die Hilfsstoffe oder die Trägersubstanzen ausgewählt sind aus der Gruppe bestehend aus Bindemitteln, Verdünnungsmitteln, Chelatbildnern, Zerfallsbeschleunigern, Verdickungs- oder Filmbildnern, Feuchthaltemitteln, Süßungsmitteln, Schleifmitteln, Aromastoffen, Maskierungsmitteln, Puffermitteln, Kühlmitteln, Farbstoffen, Tensiden, Konservierungsmitteln, Schmiermitteln, Trübungsmitteln und Lösungsmitteln.

9. Die Zusammensetzung nach einem der Ansprüche 1 bis 8, die weiterhin einen oder mehrere zusätzliche Wirkstoffe umfasst, die ausgewählt aus antimikrobiellen Mitteln, Antiplaquemitteln, antikariogenen Mitteln, parodontalen Geweberegenerationsmitteln und Hydratationsmitteln ist.

10. Die Zusammensetzung nach einem der Ansprüche 1 bis 9, die ein Mundwasser, eine Mundspülung, ein Zahnputzmittel, eine Zahnpasta, ein Zahnpulver, eine orale Lösung, ein Spray, ein Zahngel, eine Dispersion durch einen Wasserstrahl, ein Atemerfrischer oder ein Gurgelmittel ist.

11. Die Zusammensetzung nach einem der Ansprüche 1 bis 10, die ein Mundwasser oder ein Spray ist und wobei die Fluoridionenquelle in einer Menge von 0,02 bis 0,15 Gew.-% bezogen auf das Gesamtgewicht der Zusammensetzung enthalten ist.

12. Die Zusammensetzung nach einem der Ansprüche 1 bis 10, die eine Zahnpasta ist, wobei die Fluoridionenquelle in einer Menge von 0,2 bis 1 Gew.-% bezogen auf das Gesamtgewicht der Zusammensetzung enthalten ist.

13. Eine Zusammensetzung wie in einem der Ansprüche 1 bis 12 definiert zur Verwendung bei der Behandlung von Mundgeruch oder bei der Prophylaxe von Zahnkaries.

14. Eine Zusammensetzung wie in einem der Ansprüche 1 bis 12 zur Verwendung bei der Prophylaxe und/oder der Behandlung von Parodontalerkrankungen, Gingivitis oder Zahnbelag.

15. Kosmetische Verwendung einer kosmetischen Zusammensetzung wie in einem der Ansprüche 1 bis 12 definiert als Mundpflegemittel.

## Revendications

1. Une composition buccale pharmaceutique, vétérinaire ou cosmétique qui comprend une quantité efficace de :
a) octénidine ou un sel pharmaceutiquement, vétérinaire ou cosmétiquement acceptable de celle-ci,
b) une source d'ions zinc, et
c) une source d'ions fluorure,
conjointement avec un ou plusieurs excipients ou véhicules oraux appropriés pharmaceutiquement, vétérinaires ou cosmétiquement acceptables.

2. La composition selon la revendication 1, dans laquelle l'octénidine est présente comme dichlorhydrate d'octénidine.

3. La composition selon l'une quelconque des revendications 1 à 2, dans laquelle l'octénidine ou son sel est comprise en une quantité de 0,005 à 0,5 % en poids par rapport au poids total de la composition.

4. La composition selon l'une quelconque des revendications 1 à 3, dans laquelle la source d'ions zinc est choisie parmi l'acétate de zinc, le chlorure de zinc, le pidolate de zinc, le lactate de zinc et des combinaisons de ceux-ci.

5. La composition selon l'une quelconque des revendications 1 à 4, dans laquelle la source d'ions zinc est comprise en une quantité de 0,01 à 0,3 % en poids par rapport au poids total de la composition.

6. La composition selon l'une quelconque des revendications 1 à 5, dans laquelle la source d'ions fluorure est le fluorure de sodium.

7. La composition selon l'une quelconque des revendications 1 à 6, dans laquelle la source d'ions fluorure est comprise en une quantité de 0,01 à 1 % en poids par rapport au poids total de la composition.

8. La composition selon l'une quelconque des revendications 1 à 7, dans laquelle les excipients ou les véhicules sont choisis dans le groupe constitué des liants, des diluants, des agents chélants, des désintégrants, des agents épaississants ou filmogènes, des humectants, des édulcorants, des agents abrasifs, des agents aromatisants, des agents de masquage, des agents tampons, des agents de refroidissement, des agents colorants, des tensioactifs, des conservateurs, des lubrifiants, des opacifiants et des solvants.

9. La composition selon l'une quelconque des revendications 1 à 8, qui comprend en outre un ou plusieurs ingrédients actifs additionnels choisis parmi des agents antimicrobiens, des agents antiplaque, des agents anticariogènes, des agents de régénération des tissus parodontaux et des hydratants.

10. La composition selon l'une quelconque des revendications 1 à 9, qui est un bain de bouche, un rince-bouche, un dentifrice, une pâte dentifrice, une poudre dentaire, une solution orale, un spray, un gel dentaire, une dispersion au jet d'eau, un agent rafraîchisseur d'haleine ou un gargarisme.

11. La composition selon l'une quelconque des revendications 1 à 10, qui est un bain de bouche ou un spray et dans laquelle la source d'ions fluorure est comprise en une quantité de 0,02 à 0,15 % en poids par rapport au poids total de la composition.

12. La composition selon l'une quelconque des revendications 1 à 10, qui est une pâte dentifrice dans laquelle la source d'ions fluorure est comprise en une quantité de 0,2 à 1 % en poids par rapport au poids total de la composition.

13. Une composition telle que définie dans l'une quelconque des revendications 1 à 12, pour l'utilisation dans le traitement de l'halitose ou dans la prophylaxie des caries dentaires.

14. Une composition telle que définie dans l'une quelconque des revendications 1 à 12, pour l'utilisation dans la prophylaxie et / ou le traitement des maladies parodontales, de la gingivite ou de la plaque dentaire.

15. Utilisation cosmétique d'une composition cosmétique telle que définie dans l'une quelconque des revendications 1 à 12, en tant qu'agent de soin bucco-dentaire.
